# FASCICULE DE BREVET EUROPEEN

(11) **EP 1 587 500 B1**
(45) Date de publication et mention de la délivrance du brevet: **12.03.2014**
(21) Numéro de dépôt: 03799644.4
(22) Date de dépôt: 19.12.2003
(51) Int. Cl.: A61K 9/16, B01J 2/00

(54) **COMPOSITIONS POUR ADMINISTRATION ORALE DE PRINCIPES ACTIFS NECESSITANT UN MASQUAGE DU GOUT**
ARZNEIMITTEL ZUR ORALEN VERABREICHUNG VON WIRKSTOFFEN, DIE EINE GESCHMACKSVERDECKUNG BRAUCHEN
COMPOUNDS FOR ORAL DOSAGE OF ACTIVE SUBSTANCES REQUIRING A TASTE MASKING

(30) Priorité: 23.12.2002 FR 0216521
(43) Date de publication de la demande: 26.10.2005
(73) Titulaire: Aventis Pharma S.A., 92160 Antony (FR)
(72) Inventeur: CHACORNAC, Isabelle, F-38460 Saint Romain de Jalionas (FR); PROBECK, Patricia, F-69002 Lyon (FR)
(74) Mandataire: Weber, Mathieu
(86) Numéro de dépôt international: PCT/FR2003/003813
(87) Numéro de publication internationale: WO 2004/058137

(56) Documents cités:
- WO-A-00/40339
- US-A- 5 188 838
- US-A- 5 294 298
- US-A- 5 498 447
- DATABASE WPI Section Ch, Week 198434 Derwent Publications Ltd., London, GB; Class B07, AN 1984-210435 XP002312796 & JP 59 122425 A (KAKEN PHARM CO LTD) 14 juillet 1984 (1984-07-14)

## Description

La présente invention concerne des compositions destinées à l'administration orale de principes actifs de goût inacceptable, ainsi que leur préparation. Notamment la présente invention concerne des compositions pharmaceutiques.

Certains principes actifs présentent des propriétés organoleptiques inacceptables et de ce fait sont inaptes à la préparation de formulations pédiatriques ou de formulations orales destinées à des sujets dont la déglutition est difficile et peut poser des problèmes. Pour ces raisons certains produits majeurs sont privés de formulation pédiatriques et de plus certains sujets sont privés de traitement au moyen de ces principes actifs, ce qui peut avoir des conséquences extrêmement préjudiciables, voire vitales.

Le problème du masquage du goût a toujours été un problème important pour l'industrie pharmaceutique. De nombreux systèmes ont été essayés, cependant dans le cas de principes actifs trop amers les systèmes d'enrobage se sont la plupart du temps montrés insuffisants et les systèmes particulaires, lorsqu'ils ont une meilleure efficacité, présentent des inconvénients de granulométrie trop importante conduisant à un aspect sableux dans la bouche et à un refus du médicament par le patient.

Dans le brevet européen EP 639365 a été décrite une méthode pour préparer des particules enrobées, par spray coating au moyen d'une cire fondue pulvérisée par une buse bifluide. Cependant cette méthode est basée sur la pulvérisation de la cire fondue sur les particules, pour former l'enrobage. Aucun mélange du principe actif n'est effectué préalablement avec la cire, de plus les particules et la buse présentent des diamètres importants. Enfin les essais selon la méthode de la présente invention, basés uniquement sur l'utilisation d'une cire fondue, n'ont pas donné de résultats acceptables en termes de cinétique de libération à pH=1.

Il a été trouvé maintenant que des compositions destinées à l'administration orale pouvaient être mises au point et apporter un masquage de goût suffisant pour être acceptable et permettre notamment l'administration de compositions pharmaceutiques chez le jeune enfant ou chez des sujets n'étant pas en état de déglutir.

Les compositions selon l'invention comprennent de 15 à 30 % de principe actif mélangé à 60 à 80 % d'un ester de glycérol ou d'un acide gras, éventuellement additionné d'une cire, et additionné d'un agent tensio-actif et sont préparées par un procédé de spray-cooling pouvant conduire à une granulométrie inférieure à 350 µm.

De manière avantageuse, la sélection d'esters de glycérol présentant un profil de sensibilité au pH approprié, permet la libération du principe actif à des conditions de pH acides telles que l'on rencontre dans l'estomac.

Selon l'invention les esters de glycérol ou d'acide gras employés dans les compositions selon l'invention ont les caractéristiques suivantes : température de fusion comprise entre 25°C et 100°C, de préférence entre 25 et 70°C, stabilité à l'état fondu. L'ester de glycérol peut être choisi parmi le stéarate de glycérol ou le palmitostéarate de glycérol, notamment le Précirol®. L'ester de glycérol est avantageusement compris entre 50 et 85 % en poids du mélange total de la composition ; de préférence il est compris entre 60 et 80 % en poids et plus particulièrement entre 70 et 80 % en poids.

La cire pouvant être optionnellement additionnée peut être avantageusement la cire de carnauba, elle peut être également choisie parmi la paraffine ou cire d'abeille ou de candelila. Lorsqu'une cire est additionnée à la composition, elle peut être additionnée à raison de 4 à 10 % en poids du mélange total de la composition et dans un rapport de 5 à 20 % vis à vis de l'ester de glycérol introduit.

Lorsque l'on introduit un acide gras dans la composition, ce dernier est avantageusement choisi parmi l'acide palmitique, myristique ou stéarique. L'acide gras est introduit à raison de 60 à 80 % en poids du mélange total de la composition.

L'agent tensio-actif introduit dans la composition est avantageusement choisi parmi les lécithines, notamment la lécithine de soja, ou des tensio-actifs de la famille des esters de sorbitan présentant une HLB inférieure à 7. L'agent tensio-actif est additionné raison de 1 à 3 % en poids du mélange total de la composition.

De préférence, les diamètres sont avantageusement inférieurs à 350 µm pour plus de 90% des particules. Plus particulièrement ils peuvent être compris entre 100 µm et 350 µm pour 25 à 65 % des particules et inférieurs à 100 µm pour 35 à 75% des particules.

Selon l'invention, le spray-cooling est effectué par pulvérisation au moyen d'une buse bifluide permettant d'assurer l'obtention de la granulométrie souhaitée, c'est à dire une granulométrie de faible diamètre tel que décrit ci-dessus.

Selon l'invention, la préparation de la composition s'effectue par mélange du principe actif dans l'ester de glycérol fondu, additionné des autres excipients. Le mélange est pulverisé par la buse bifluide en haut d'une tour dans laquelle est éventuellement introduit un contre courant gazeux froid destiné à aider à la solidification des goutelettes pulvérisées. Le dispositif est de préférence muni d'un lit fluidisé permettant de récupérer les particules et d'améliorer la rapidité de solidification.

Le mélange fondu introduit dans la buse bifluide est généralement chauffé entre 60 et 100°C.
De préférence la buse bifluide comprend avantageusement un diamètre de 2,5 mm pour la section liquide et une section torrique de 0,3 mm pour la section d'air (ou d'azote). Le débit de liquide et le débit d'air (ou d'azote) pulvérisés dans la buse sont fixés préalablement en fonction des diamètres des sections de la buse bifluide utilisée. De préférence le débit de liquide est fixé entre 1 et 15 kg/h et le débit d'air est fixé entre 2 et 5 m³/h.

La granulométrie du principe actif mélangé initialement à l'ester de glycérol s'étale de 2 à 350 µm. Dans certains cas il peut être nécessaire de procéder à un broyage avant ou après le mélange avec l'ester de glycérol et préalablement à la pulvérisation. De préférence on effectue le broyage à sec préalablement au mélange.
La tour utilisée est une tour du type tour de prilling, mais à laquelle a été adaptée une buse bifluide (contrairement à la mise en oeuvre habituelle du prilling). La hauteur de la tour est de préférence comprise entre 2 et 8 m. Le contre-courant gazeux destiné au refroidissement est avantageusement un courant d'azote ou un courant de gaz sec. Le débit est fonction de nombreux facteurs tels que les températures, la hauteur de chambre, les quantités de produit... A titre indicatif il peut être fixé notamment entre des valeurs : légèrement supérieure à 0 et 350 Nm3/h.

La composition peut en outre contenir d'autres additifs tels que des agents édulcorants ou modifiant le goût (saccharinate, aspartame, glycérine, vanilline, menthol ...ou toute autre substance habituellement utilisée dans l'industrie pharmaceutique), des arômes, des agents d'écoulement, des lubrifiants, des ballasts ou des agents minéraux [silices, oxyde d'aluminium, oxyde de magnésium, talc ... carbonates (carbonate de calcium), phosphates (phosphate tricalcique), lactose, sorbitol, glycocolle, mannitol, glucose, maltodextrines ...], des agents conservateurs (à titre d'exemple métabisulfite de sodium, propylène glycol, éthanol ou glycérine), des agents destinés à modifier la couleur. De préférence il s'agit d'une composition pharmaceutique.

La présente invention concerne tous les principes actifs seuls ou en mélanges, administrables par voie orale et présentant des problèmes organoleptiques, ayant pour conséquence une inacceptabilité par les personnes devant les ingérer. Les principes actifs sont des substances amères, irritantes... ou de saveur inacceptable. Lesdits principes actifs sont compatibles avec l'ester de glycérol et sa température de fusion.
A titre non limitatif, lorsqu'il s'agit de principes actifs pharmaceutiques, ils peuvent appartenir à toutes classes thérapeutiques, comme par exemple les antibactériens [macrolides (spiramycine, kétolides comme par exemple la telithromycine...), streptogramines (pristinamycines comme la pyostacine par exemple, virginiamycine par exemple), quinolones...], les antifongiques (metronidazole...), les antiparasitaires (nivaquine...), les anti-viraux, les anticancéreux, les analgésiques, les anti-inflammatoires non stéroïdiens, les antitussifs, les psychotropes, les stéroïdes, les médicaments destinés au traitement des allergies, les antiasthmatiques, les antispasmodiques, les cardiovasculaires (roxitromycine par exemple...) les agents thérapeutiques du tractus gastro intestinal...
Il peut également s'agir de principes actifs, seuls ou en mélanges, à destination cosmétique comme des vitamines ou des extraits de végétaux ou animaux

La présente invention présente l'avantage d'un masquage de goût efficace allié à une absence ou à une très faible sensation sablonneuse de la composition dans la bouche.

Des tests de dissolution ont été effectués et attestent d'une faible dissolution à pH neutre donc d'un masquage de goût convenable et une dissolution à des taux de 80 à 100 % à pH =1 après 60 minutes, attestant de la libération du principe actif en milieu gastrique.

La limite d'amertume, en fonction de la nature du principe actif, est mesurée. Les essais de dissolution sont mis en oeuvre notamment dans un test de dissolution à pH neutre : test du verre d'eau, à des concentrations de 250 ou 500 mg/l. Les résultats sont appréciés au regard de la valeur limite d'amertume évaluée. Il est observé une dissolution environ 4 fois plus lente à pH neutre qu'à pH=1.

La cinétique de dissolution à pH=1 est mesurée pour des solutions de concentration 500 mg/l, en milieu HCl 0,1 N, dans un milieu de dissolution contenant 0,2 % de lauryl sulfate de sodium.

Les exemples suivants, donnés à titre non limitatif, illustrent la présente invention.

### Exemple 1

On introduit 2400 g de précirol préalablement fondu en étuve à 60°C dans un réacteur double enveloppé dont la température de consigne de double enveloppe est fixée à 75°C. On ajoute 78 g de lécithine de soja. Lorsque la lécithine de soja est dissoute, on baisse la température de consigne à 65°C et on ajoute 540 g de pristinamycine. On agite 20 minutes à 300 tr/min puis on passe la suspension sur un broyeur à billes.

1248 g de la suspension broyée sont ensuite pulvérisés par l'intermédiaire d'une buse bifluide dans une tour de prilling préalablement refroidie par un courant d'azote froid. En début de pulvérisation, la température est de 0°C en haut de tour et de -20°C en bas de tour. La pression d'air sur la buse bi-fluide est de 1,5 bars, ce qui conduit à un débit d'air de pulvérisation de 2,3 m³/h. Le débit de liquide est de 4,7 kg/h.

En fin de pulvérisation, le produit est ensuite fluidisé pendant 20 minutes à -20°C, puis pendant 2 heures à 32°C.

La granulométrie du produit obtenu, mesurée par tamisage, est de :
- 26 % de particules comprise entre 0 et 100 µm
- 62 % de particules comprise entre 100 et 315 µm
- 12 % de particules comprise entre 315 et 500 µm
- 1 % de particules supérieures à 500 µm

La cinétique de dissolution à pH 1 est de 92 % en 60 minutes pour le produit brut et 99 % en 60 minutes pour la coupe granulométrique 100-315 µm.

La concentration en matière active dans le verre d'eau (pH neutre) est de 182 mg/l après 5 minutes et 473 mg/l après 15 minutes pour les granulés bruts. Elle est de 89 mg/l après 5 minutes et 280 mg/l après 15 minutes pour les granulés de la coupe 100-315 µm.

### Exemple 2

On introduit 704 g de précirol dans un réacteur double enveloppé dont la température de consigne de double enveloppe est fixée à 75°C. Lorsque le précirol est fondu, on ajoute 18 g de lécithine de soja. Lorsque la lécithine de soja est dissoute, on ajoute 182 g de pristinamycine préalablement micronisée dans un microniseur à jet d'air et présentant après broyage un diamètre médian de 2 µm. On agite pendant 45 minutes à 800 tr/min pour obtenir une suspension homogène.

La suspension est ensuite pulvérisée par l'intermédiaire d'une buse bifluide dans une tour de prilling préalablement refroidie par un courant d'azote froid. En début de pulvérisation, la température est de -14°C en haut de tour et de -42°C en bas de tour. La pression d'air sur la buse bi-fluide est de 1,5 bars, ce qui conduit à un débit d'air de pulvérisation de 2,3 m³/h. Le débit de liquide est de 10,8 kg/h.

La granulométrie du produit obtenu, mesurée par tamisage, est de :
- 30 % de particules comprise entre 0 et 100 µm
- 54 % de particules comprise entre 100 et 315 µm
- 11 % de particules comprise entre 315 et 500 µm
- 5 % de particules supérieures à 500 µm

La cinétique de dissolution à pH 1 pour le produit brut est de 86 % en 60 minutes et 97 % en 120 minutes.

La concentration en matière active dans le verre d'eau (pH neutre) est de 22 mg/l après 5 minutes et 140 mg/l après 15 minutes pour les granulés bruts.

### Exemple 3

On introduit 907 g de précirol dans un réacteur double enveloppé dont la température de consigne de double enveloppe est fixée à 70°C. Lorsque le précirol est fondu, on ajoute 23 g de lécithine de soja. Lorsque la lécithine de soja est dissoute, on introduit 207 g de télithromycine non broyée et présentant un diamètre médian de 114 µm. On agite pendant 50 minutes à 500 tr/min pour obtenir un liquide homogène : la télithromycine est visiblement soluble dans le précirol.

La suspension est ensuite pulvérisée par l'intermédiaire d'une buse bifluide dans une tour de prilling préalablement refroidie par un courant d'azote froid. En début de pulvérisation, la température est de 0°C en haut de tour et de -20°C en bas de tour. La pression d'air sur la buse bi-fluide est de 1,3 bars, ce qui conduit à un débit d'air de pulvérisation de 4 m³/h. Le débit de liquide est de 8,5 kg/h.

La granulométrie du produit obtenu, mesurée par tamisage, est de :
- 59 % de particules comprise entre 0 et 100 µm
- 38 % de particules comprise entre 100 et 315 µm
- 3 % de particules comprise entre 315 et 500 µm

La cinétique de dissolution à pH 1 pour le produit brut est de 98 % en 60 minutes.

La concentration en matière active dans le verre d'eau (pH neutre) est de 387 mg/l après 5 minutes et 873 mg/l après 15 minutes pour les granulés bruts. Elle est de 181 mg/l après 5 minutes et 475 mg/l après 15 minutes pour les granulés de la coupe 100-315 µm.

### Exemple 4

On introduit 782 g de précirol et 115 g de cire de carnauba dans un réacteur double enveloppé dont la température de consigne de double enveloppe est fixée à 95°C. Lorsque les corps gras sont fondus, on ajoute 23 g de lécithine de soja. Lorsque la lécithine de soja est dissoute, on introduit 230 g de télithromycine non broyée et présentant un diamètre médian de 114 µm. On agite pendant 60 minutes à 500 tr/min pour obtenir un liquide homogène.

La suspension est ensuite pulvérisée par l'intermédiaire d'une buse bifluide dans une tour de prilling préalablement refroidie par un courant d'azote froid. En début de pulvérisation, la température est de -7°C en haut de tour et de -29°C en bas de tour.

La pression d'air sur la buse bi-fluide est de 1,3 bars, ce qui conduit à un débit d'air de pulvérisation de 4 m³/h. Le débit de liquide est de 5 kg/h.

La granulométrie du produit obtenu, mesurée par tamisage, est de :
- 27 % de particules comprise entre 0 et 100 µm
- 50 % de particules comprise entre 100 et 315 µm
- 16 % de particules comprise entre 315 et 500 µm
- 7 % de particules supérieures à 500 µm

La cinétique de dissolution à pH 1 pour le produit brut est de 77,5 % en 60 minutes.

La concentration en matière active dans le verre d'eau (pH neutre) est de 90 mg/l après 5 minutes et 340 mg/l après 15 minutes pour les granulés bruts. Elle est de 81 mg/l après 5 minutes et 658 mg/l après 15 minutes pour les granulés de la coupe 100-315 µm.

## Revendications

1. Une composition destinée à l'administration orale de principes actifs de goût inacceptable, **caractérisée en ce qu'**elle comprend de 15 à 30 % de principe actif mélangé à 50 à 85 % d'un ester de glycérol ou d'un acide gras, éventuellement additionné d'une cire, et additionné d'un agent tensio-actif qui est choisi parmi les lécithines et **en ce qu'**elle est préparée par un procédé de spray-cooling conduisant à une granulométrie inférieure à 350 µm pour plus de 90% des particules.

2. Une composition selon la revendication 1, **caractérisée en ce que** l'ester de glycérol est choisi parmi le stéarate de glycérol ou le palmitostéarate de glycérol.

3. Une composition selon la revendication 1, **caractérisée en ce que** l'ester de glycérol est compris entre 60 et 80 % en poids du mélange total de la composition.

4. Une composition selon l'une des revendications 1 à 3, **caractérisée en ce que** l'ester de glycérol est compris entre 70 et 80 % en poids du mélange total de la composition.

5. Une composition selon l'une des revendications 1 à 4, **caractérisée en ce que** la lécithine est de la lécithine de soja.

6. Une composition selon l'une des revendications 1 à 5, **caractérisée en ce que** le principe actif est un principe actif pharmaceutique.

7. Un procédé de masquage de gout de composition à administration par voie orale contenant des principes actifs au gout inacceptable comprenant la préparation d'une composition selon l'une des revendications 1 à 5, **caractérisé en ce que** l'on mélange le principe actif dans l'ester de glycérol fondu, additionné des autres excipients, puis met en oeuvre un procédé de spray-cooling par pulvérisation au moyen d'une buse bifluide en haut d'une tour dans laquelle est éventuellement introduit un contre courant gazeux froid.

8. Un procédé de préparation selon la revendication 7, **caractérisé en ce que** le dispositif est muni d'un lit fluidisé.

9. Une composition selon la revendication 1 **caractérisée en ce que** l'acide gras est choisi parmi l'acide stéarique, l'acide myristique ou l'acide palmitique.

## Patentansprüche

1. Zusammensetzung zur oralen Verabreichung von Wirkstoffen mit unannehmbarem Geschmack, **dadurch gekennzeichnet, dass** sie 15 bis 30% Wirkstoff in Abmischung mit 50 bis 85% eines Glycerin- oder Fettsäureesters, gegebenenfalls unter Zusatz eines Wachses, und unter Zusatz eines Tensids, das aus Lecithinen ausgewählt ist, umfasst und dass sie durch ein Sprühkühlverfahren, das zu einer Teilchengröße von weniger als 350 µm für mehr als 90% der Teilchen führt, hergestellt wird.

2. Zusammensetzung nach Anspruch 1, **dadurch gekennzeichnet, dass** der Glycerinester aus Glycerinstearat oder Glycerinpalmitostearat ausgewählt ist.

3. Zusammensetzung nach Anspruch 1, **dadurch gekennzeichnet, dass** der Glycerinester zwischen 60 und 80 Gew.-% der gesamten Mischung der Zusammensetzung ausmacht.

4. Zusammensetzung nach einem der Ansprüche 1 bis 3, **dadurch gekennzeichnet, dass** der Glycerinester zwischen 70 und 80 Gew.-% der gesamten Mischung der Zusammensetzung ausmacht.

5. Zusammensetzung nach einem der Ansprüche 1 bis 4, **dadurch gekennzeichnet, dass** es sich bei dem Lecithin um Sojalecithin handelt.

6. Zusammensetzung nach einem der Ansprüche 1 bis 5, **dadurch gekennzeichnet, dass** es sich bei dem Wirkstoff um einen pharmazeutischen Wirkstoff handelt.

7. Verfahren zum Maskieren des Geschmacks einer Zusammensetzung zur Verabreichung auf oralem Wege, die Wirkstoffe mit unannehmbarem Geschmack enthält, bei dem man eine Zusammensetzung nach einem der Ansprüche 1 bis 5 herstellt, **dadurch gekennzeichnet, dass** man den Wirkstoff mit dem geschmolzenen Glycerinester mischt, die anderen Hilfsstoffe zugibt und dann ein Sprühkühlverfahren durch Sprühen mit Hilfe einer Zweifluiddüse am Kopf eines Turms, in den gegebenenfalls ein kalter gasförmiger Gegenstrom eingeleitet wird, durchführt.

8. Herstellungsverfahren nach Anspruch 7, **dadurch gekennzeichnet, dass** die Vorrichtung mit einer Wirbelschicht ausgestattet ist.

9. Zusammensetzung nach Anspruch 1, **dadurch gekennzeichnet, dass** es sich bei der Fettsäure um Stearinsäure, Myristinsäure oder Palmitinsäure handelt.

## Claims

1. A composition intended for oral administration of active ingredients with unacceptable taste, **characterized in that** it comprises from 15 to 30% of active ingredient mixed with 50 to 85% of an ester of glycerol or a fatty acid optionally supplemented with wax, and supplemented with a surfactant which is chosen from lecithins, and **in that** it is prepared by a spray-cooling process which leads to a particle size of less than 350 µm for more than 90% of the particles.

2. The composition of claim 1, **characterized in that** the glycerol ester is chosen from glyceryl stearate or glyceryl palmitostearate.

3. The composition of claim 1, **characterized in that** the glycerol ester is between 60 and 80% by weight of the total mixture of the composition.

4. The composition of one of claims 1 to 3, **characterized in that** the glycerol ester is between 70 and 80% by weight of the total mixture of the composition.

5. The composition of one of claims 1 to 4, **characterized in that** the lecithin is soybean lecithin.

6. The composition of one of claims 1 to 5, **characterized in that** the active ingredient is a pharmaceutical active ingredient.

7. A method for masking the taste of a composition for oral administration containing active ingredients with unacceptable taste, comprising the preparation of a composition of one of claims 1 to 5, **characterized in that** the active ingredient is mixed with the molten glycerol ester, supplemented with other excipients, and then a spray-cooling process is performed by spraying by means of a dual-fluid nozzle at the top of a tower into which a cold gaseous stream is optionally introduced countercurrentwise.

8. The process of preparation of claim 7, **characterized in that** the device is provided with a fluidized bed.

9. A composition of claim 1, **characterized in that** the fatty acid is chosen from stearic acid, myristic acid or palmitic acid.
